# EUROPÄISCHE PATENTSCHRIFT

(11) **EP 2 446 882 B1**
(45) Veröffentlichungstag und Bekanntmachung des Hinweises auf die Patenterteilung: **18.12.2013**
(21) Anmeldenummer: 10189170.3
(22) Anmeldetag: 28.10.2010
(51) Int. Cl.: A61K 9/50, A61K 31/485

(54) **Arzneimittel mit dem Wirkstoff Hydromorphon mit verbesserter Lagerstabilität**
Medicament with improved storage stability containing the active ingredient hydromorphone
Médicament doté de l'agent actif hydromorphone à stabilité en stockage améliorée

(43) Veröffentlichungstag der Anmeldung: 02.05.2012
(73) Patentinhaber: Acino Pharma AG, 4058 Basel (CH)
(72) Erfinder: Zingraff, Marc, 68300, Saint-Louis (FR); Lütolf, Walter, 4312, Magden (CH)
(74) Vertreter: Best, Michael

(56) Entgegenhaltungen:
- EP-A1- 1 444 977
- EP-A1- 2 057 984
- US-A1- 2003 073 714
- US-A1- 2010 028 389

## Beschreibung

Die vorliegende Erfindung betrifft Arzneimittel mit einem oder mehreren wirkstoffhaltigen Teilchen zur Verabreichung des Wirkstoffs Hydromorphon. Die Teilchen retardieren die Wirkstofffreisetzung. Die erfindungsgemäßen Arzneimittel und Teilchen weisen eine hervorragende Lagerstabilität auf.

Arzneimittel mit dem Wirkstoff Hydromorphon sind schon seit langem bekannt. Es ist ebenfalls bekannt, Hydromorphon über Retardformulierungen, bei denen der Wirkstoff langsam über einen längeren Zeitraum hinweg mit einem bestimmten Freisetzungsprofil freigesetzt wird, zu verabreichen. Derartige Arzneimittel sind beispielsweise schon in der EP-A 0 271 193 beschrieben. Diese Druckschrift offenbart beispielhaft Tabletten, bei denen Hydromorphonhydrochlorid in einer retardierenden Matrix formuliert ist. Die Druckschrift offenbart ebenfalls in allgemeiner Form, dass das Arzneimittel als Teilchen vorliegen kann, das mit einer die Freisetzung kontrollierenden Filmbeschichtung versehen ist. Die Filmbeschichtung wird so gewählt, dass ein bestimmtes *in vitro* Freisetzungsprofil erzielt wird. Die Druckschrift offenbart keine Stabilitätsprobleme für den Wirkstoff Hydromorphonhydrochlorid, Antioxidationsmittel werden nicht erwähnt.

Die EP-A 0 548 448 offenbart allgemein, dass es bei Retardformulierungen, bei denen der Wirkstoff als Beschichtung auf einem inerten Kern vorliegt, zu Stabilitätsproblemen kommen kann, wenn die Beschichtung aus einem wässrigen System heraus aufgetragen wird. Als Beispiele für derartige Retardformulierungen werden auch Formulierungen mit dem Wirkstoff Hydromorphon angegeben und die meisten Beispiele der Druckschrift betreffen Hydromorphon. Zur Lösung dieser Stabilitätsprobleme schlägt die Druckschrift vor, die beschichteten Kerne einer besonderen Härtungsreaktion zu unterziehen. Antioxidationsmittel werden nicht erwähnt.

Die US 5,866,164 offenbart Arzneimittel mit einem Opioid und einem Opioidantagonisten. Die Arzneimittel weisen zwei Schichten auf, eine Schicht, in der das Hydromorphon vorliegt und eine Schicht, die den Opioidantagonisten enthält. Zusammen mit dem Opioidantagonisten kann ein Antioxidationsmittel formuliert sein. Von Stabilitätsproblemen des Hydromorphons wird in der Druckschrift nicht berichtet, das Hydromorphon wird auch nicht mit einem Antioxidationsmittel formuliert.

Die US 2008/0020032 offenbart ein Verfahren, um Arzneimittel mit Opioiden, unter anderem auch Hydromorphon, gegen Missbrauch zu sichern. Von Stabilitätsproblemen des Hydromorphons in den Arzneimitteln wird nicht berichtet. In keinem der Beispiele der Druckschrift wird Hydromorphon mit einem Antioxidationsmittel zusammen formuliert.

Eine ähnliche Offenbarung kann auch den Druckschriften US 2007/0104789 und US 2010/0047345 entnommen werden. In keiner dieser Druckschriften wird von Stabilitätsproblemen des Hydromorphons während der Lagerung eines Arzneimittels berichtet, und in keiner dieser Druckschriften wird Hydromorphon mit einem Antioxidationsmittel formuliert.

Die US 5,914,131 und die US 2010/0028389 betreffen Arzneimittel mit dem Wirkstoff Hydromorphon, bei denen es sich um osmotische Systeme handelt. Wie aus der Figur 3 dieser Druckschriften hervorgeht, befinden sich die Wirkstoffzusammensetzungen und eine expandierende Zusammensetzung in einer doppelschichtigen Anordnung. Die expandierende Zusammensetzung enthält ein Osmopolymer, ein osmotisches Mittel (Osmagent) und gegebenenfalls ein Antioxidationsmittel. Von Stabilitätsproblemen des Hydromorphons bei der Lagerung des Arzneimittels wird nicht berichtet, und Hydromorphon wird auch nicht im Gemisch mit einem Antioxidationsmittel formuliert.

Die WO 2009/059701 offenbart Retardtabletten mit dem Wirkstoff Hydromorphon. Die Retardtabletten enthalten Pellets, bei denen ein inerter Kern mit einer Wirkstoffschicht, die das Hydromorphon enthält, beschichtet ist. Auf diese Beschichtung wird eine retardierende Beschichtung aufgebracht, und auf diese Art und Weise wird ein Zwischenprodukt zur Herstellung des finalen Produkts hergestellt. Aus dem Zwischenprodukt wird das finale Produkt dadurch hergestellt, dass auf der retardierenden Beschichtung nochmals eine schnell freisetzende Beschichtung mit dem Wirkstoff Hydromorphon aufgebracht wird. Die so hergestellten Pellets können insbesondere zu sogenannten "MUPS"-Tabletten verpresst werden. Auch in der WO 2009/059701 findet sich kein Hinweis auf eine mögliche Instabilität des Hydromorphons bei der Lagerung, und das Hydromorphon wird nicht mit einem Antioxidationsmittel zusammen formuliert.

Die Erfinder der vorliegenden Anmeldung haben nunmehr gefunden, dass bei den im Stand der Technik beschriebenen Arzneimitteln mit dem Wirkstoff Hydromorphon im Kern und einer darauf aufgebrachten Retardbeschichtung bei der Lagerung ein Stabilitätsproblem auftritt. Insbesondere kommt es gerade in den ersten Wochen nach der Herstellung entsprechender Arzneimittel zu einer unerwünschten Zersetzung des Wirkstoffs.

Aufgabe der Erfindung war es, ein Arzneimittel zur Verfügung zu stellen, das eine wesentlich verbesserte Lagerstabilität aufweist und bei dem es insbesondere auch in den ersten Wochen der Lagerung nicht zu einer Wirkstoffzersetzung kommt.

Diese Aufgabe wird durch den Gegenstand der Patentansprüche gelöst.

Die Lösung der Aufgabe beruht auf dem überraschenden Befund, dass, anders als im Stand der Technik angenommen, Hydromorphon sowie die Salze davon und Solvate davon in Retardformulierungen die beschichtete Teilchen mit wirkstoffhaltigem Kern enthalten oxidationsempfindlich sind. Nachdem überraschenderweise gefunden wurde, dass die geringe Lagerstabilität des Hydromorphons in den bekannten Formulierungen auf eine Empfindlichkeit des Hydromorphons gegenüber Luftsauerstoff zurückzuführen ist, konnte das Stabilitätsproblem dadurch gelöst werden, dass man Formulierungen zur Verfügung stellt, bei denen das Hydromorphon mit einem Antioxidationsmittel gemischt im Kern eines Teilchens mit einer Retardbeschichtung vorlegt. Hierdurch werden Arzneimittel mit hervorragender Lagerstabilität zur Verfügung gestellt.

Die vorliegende Erfindung stellt in einer bevorzugten Ausführungsform ein wirkstoffhaltiges Teilchen mit retardierter Wirkstofffreisetzung zur Verfügung, welches folgenden Aufbau aufweist:
a) ein inertes Pellet,
b) eine auf dem inerten Pellet aufgebrachte Wirkstoffschicht, die den Wirkstoff im Gemisch mit dem Antioxidationsmittel enthält und,
c) eine auf die Wirkstoffschicht aufgebrachte Retardbeschichtung, die die Freisetzung des Wirkstoffs retardiert und die sich bevorzugt direkt auf der Wirkstoffschicht befindet,
wobei die Wirkstoffschicht das Hydromorphon oder das pharmazeutisch verträgliche Salz davon oder das Solvat davon im Gemisch mit dem Antioxidationsmittel Ascorbinsäure oder ein Salz oder ein Ester davon enthält.

Bei einer alternativen bevorzugten Ausführungsform weist das wirkstoffhaltige Teilchen einen Kern auf, bei dem es sich um ein Extrusionspellet handelt, in dem der Wirkstoff im Gemisch mit einem Sphäronisierungsmittel und einem Antioxidationsmittel vorliegt. Der Kern ist mit einer Retardbeschichtung beschichtet, die die Freisetzung des Wirkstoffs retardiert.

Die Erfindung stellt ebenfalls ein Arzneimittel zur oralen Verabreichung mit mindestens einem, bevorzugt mehreren derartiger wirkstoffhaltiger Teilchen zur Verfügung sowie auch ein Verfahren zur Herstellung der Teilchen und die Verwendung der Arzneimittel zur Behandlung chronischer Schmerzen.

Die erfindungsgemäßen Teilchen und damit auch das erfindungsgemäße Arzneimittel enthalten als Wirkstoff Hydromorphon. Bevorzugt ist Hydromorphon der einzige Wirkstoff, der in den erfindungsgemäßen Teilchen und dem erfindungsgemäßen Arzneimittel vorhanden ist. Der Wirkstoff ist in dem erfindungsgemäßen Arzneimittel bevorzugt in einer Konzentration im Bereich von 0,5 Gew.-% bis 20 Gew.-%, insbesondere im Bereich von 1 Gew.-% bis 10 Gew.-%, bezogen auf das Gesamtgewicht des Arzneimittels enthalten, besonders bevorzugt im Bereich von 1,5 Gew.-% bis 5,0 Gew.-%. Bevorzugt enthält das erfindungsgemäße Arzneimittel Hydromorphon bzw. das Salz oder Solvat davon im Bereich von 1 bis 100 mg, insbesondere im Bereich von 2 bis 50 mg, stärker bevorzugt im Bereich von 2 bis 40 mg, beispielsweise im Bereich von 2 mg bis 30 mg, am stärksten bevorzugt im Bereich von 2 mg bis 24 mg.

Erfindungsgemäß bevorzugt weist ein wirkstoffhaltiges Teilchen einen Gehalt an Wirkstoff im Bereich von 1 Gew.-% bis 40 Gew.-%, stärker bevorzugt im Bereich von 2 Gew.-% bis 30 Gew.-% insbesondere im Bereich von 2 Gew.-% bis 10 Gew.-% auf, bezogen auf das Gewicht des Kerns, der den Wirkstoff enthält (z.B. inertes Pellet + Wirkstoffschicht bzw. Extrusionspellet).

Der Wirkstoff liegt bevorzugt als Hydrochlorid vor, er kann aber auch als ein anderes Salz oder als Solvat oder als Solvat eines Salzes oder als freie Base, vorliegen. Wenn im Rahmen dieser Anmeldung von dem Wirkstoffgehalt gesprochen wird, bezieht sich dieser immer auf das Gewicht des Salzes oder des Solvats, falls ein Salz oder Solvat eingesetzt wird. Unter einem Solvat des Wirkstoffes wird auch ein Solvat des Salzes des Wirkstoffes verstanden.

Als Antioxidationsmittel kann ein beliebiges bekanntes pharmazeutisch verträgliches Antioxidationsmittel verwendet werden, z.B. Ascorbinsäure, butyliertes Hydroxyanisol, butyliertes Hydroxytoluol, Vitamin E, Sorbinsäure, Isoascorbinsäure, Zitronensäure etc. Bevorzugt ist das Antioxidationsmittel erfindungsgemäß wasserlöslich. Bei dem erfindungsgemäß besonders bevorzugten Antioxidationsmittel handelt es sich um Ascorbinsäure, insbesondere um natürlich vorkommende L-(+)-Ascorbinsäure (Vitamin C). Salze der Ascorbinsäure, beispielsweise Calciumascorbat oder auch Ester der Ascorbinsäure wie Ascorbylpalmitat werden ebenfalls als Antioxidationsmittel erfindungsgemäß bevorzugt.

Erfindungsgemäß bevorzugt weist das Arzneimittel einen Gehalt an Antioxidationsmittel im Bereich 0,1 bis 5 mg, stärker bevorzugt im Bereich von 0,1 bis 3 mg, insbesondere im Bereich von 0,1 bis 2 mg auf.

Erfindungsgemäß bevorzugt weist ein wirkstoffhaltiges Teilchen einen Gehalt an Antioxidationsmittel im Bereich von 0,01 Gew.-% bis 30 Gew.-%, stärker bevorzugt im Bereich von 0,05 Gew.-% bis 20 Gew.-%, insbesondere im Bereich von 0,05 Gew.-% bis 10 Gew.-%, wie beispielsweise im Bereich von 0,05 Gew.-% bis 0,5 Gew.-% auf, bezogen auf das Gewicht des Kerns (z.B. inertes Pellet + Wirkstoffschicht bzw. Extrusionspellet).

Die vorstehenden Mengenangaben für das Antioxidationsmittel beziehen sich auf die Menge an Antioxidationsmittel in den Teilchen.

Erfindungsgemäß bevorzugt ist in dem Arzneimittel und damit auch in dem wirkstoffhaltigen Teilchen das Gewichtsverhältnis von Hydromorphon bzw. dem Salz oder Solvat davon zu dem Antioxidationsmittel im Bereich von 100:1 bis 1:1, stärker bevorzugt im Bereich von 70:1 bis 5:1, noch stärker bevorzugt im Bereich von 40:1 bis 5:1.

Bei einer erfindungsgemäß bevorzugten Ausführungsform handelt es sich bei dem Kern des Teilchens um ein inertes Pellet, das eine Wirkstoffschicht aufweist.

Bei dieser Ausführungsform weisen die erfindungsgemäßen wirkstoffhaltigen Kerne ein inertes Pellet auf. Derartige inerte Pellets sind im Stand der Technik bekannt und werden beispielsweise als Non-Pareil in verschiedenen Größen vertrieben. Als Beispiel kann hier das Produkt Non-Pareil 18-20 (mesh) benannt werden. Allgemein weisen derartige inerte Pellets einen Durchmesser im Bereich von 0,2 mm bis 2,5 mm, insbesondere im Bereich von 0,2 mm bis 1,5 mm auf. Sie sind auch unter der Bezeichnung "Neutralkerne" bekannt. Häufig werden Zuckerkerne oder Kerne aus mikrokristalliner Cellulose als Neutralkerne verwendet, aber auch andere Neutralkerne sind in der Fachwelt bekannt.

Erfindungsgemäß befindet sich auf den inerten Pellets eine Wirkstoffschicht, bei der der Wirkstoff, das heißt das Hydromorphon bzw. das Salz oder Solvat davon im Gemisch mit einem Antioxidationsmittel und mit einem oder mehreren Bindemitteln als Beschichtung auf den inerten Kern aufgebracht ist. Diese Beschichtung ist bevorzugt nicht retardierend, das heißt aus ihr wird der Wirkstoff schnell freigesetzt, das heißt mindestens 90% innerhalb von 15 Minuten, bestimmt nach der Paddle-Methode der U.S.-Pharmakopöe (100 rpm in 900 ml wässrigem Puffer, pH im Bereich von 1,6 und 7,2 bei 37°C). Sofern nichts anderes angegeben ist, beziehen sich alle in dieser Anmeldung genannten Freisetzungsdaten auf *in vitro* Freisetzungen, die nach diesem Verfahren der U.S.-Pharmakopöe erhalten werden.

Diese wirkstoffhaltige Schicht, die sich auf dem inerten Pellet befindet, wird im Rahmen dieser Anmeldung als Wirkstoffschicht bezeichnet. Die Wirkstoffschicht enthält in der Regel ein Bindemittel, den Wirkstoff im Gemisch mit einem Antioxidationsmittel und kann noch weitere übliche pharmazeutisch verträgliche Hilfs- und Zusatzstoffe enthalten. Derartige Stoffe sind dem Fachmann bekannt.

Geeignete Bindemittel sind beispielsweise niedrigviskose wasserlösliche Polymere, insbesondere wasserlösliche Hydroxyniedrigalkylcellulosen wie Hydroxypropylcellulose, niedrig substituierte Hydroxypropylcellulose, Hydroxypropylmethylcellulose etc. Weitere geeignete Bindemittel sind Aminoalkylmethacrylat Copolymere, Gelatine, Gummi Arabicum, Guarkernmehl (Guar Gum), Methylcellulose, Carboxymethylcellulose, Ethylhydroxyethylcellulose, Hydroxyethylmethylcellulose, Hydroxyethylcellulose, Gum Tragant, Polyvinylpyrrolidon, Polyvinylacetat, Polyvinylalkohol sowie anorganische Gele, aber auch Dextrin, Natriumalginat, Pektin etc. Erfindungsgemäß bevorzugt handelt es sich bei dem Bindemittel um Hydroxypropylcellulose oder Hydroxypropylmethylcellulose.

Die Wirkstoffschicht kann ebenfalls beispielsweise Farbstoffe, Weichmacher wie Triethylcitrat, Polyethylenglykol oder weitere Hilfs- und Zusatzstoffe enthalten.

Bei einer zweiten erfindungsgemäß bevorzugten Ausführungsform handelt es sich bei dem Kern des erfindungsgemäßen Teilchens um ein Extrusionspellet. Das Extrusionspellet enthält den Wirkstoff, ein Antioxidationsmittel, ein Sphäronisierungsmittel und gegebenenfalls weitere pharmazeutisch verträgliche Hilfs- und Zusatzstoffe. Derartige Extrusionspellets sind dem Fachmann bekannt. Sie enthalten ein Sphäronisierungsmittel, da die Extrusionspellets nach der Extrusion noch gerundet werden, bevor sie mit einer Retardbeschichtung versehen werden. Das Sphäronisierungsmittel verleiht dem Extrusionspellet die erforderliche Plastizität, so dass es während des Rundungsvorgangs nicht zerbricht oder anderweitig zerstört wird. Das bevorzugte Sphäronisierungsmittel ist mikrokristalline Cellulose. Die Extrusionspellets bezeichnet man auch als Sphäroide.

Der Gehalt an Sphäronisierungsmittel, insbesondere der mikrokristallinen Cellulose in dem Extrusionspellet ist bevorzugt im Bereich von 40-90 Gew.-%, insbesondere von 60-85 Gew.-%, stärker bevorzugt von 65-80 Gew.-%

Neben dem Sphäronisierungsmittel kann das Extrusionspellet noch weitere übliche Hilfs-und Zusatzstoffe aufweisen, wie es im Stand der Technik bekannt ist, insbesondere ein oder mehrere Bindemittel wie Hydroxypropylmethylcellulose, Hydroxypropylcellulose und Povidon und gegebenenfalls Füllmittel wie Mannit, Sucrose, Kollidon etc. Die Bindemittel und Füllmittel sind üblicherweise wasserlöslich.

Die Extrusionspellets weisen bevorzugt einen Formfaktor von ≤ 1,4 auf. Der Formfaktor gibt die Abweichung eines Teilchens von einer Kugelgestalt wieder. Er ist definiert als Umfang x Umfang / (4 π x Fläche) wobei sich Umfang und Fläche auf einen Querschnitt des Teilchens beziehen. Bevorzugt ist der Formfaktor ≤ 1,3, stärker bevorzugt ≤ 1,2, insbesondere ≤ 1,1. Bei einer idealen Kugel beträgt der Formfaktor 1.

Erfindungsgemäß befindet sich auf dem Kern des Teilchens die Retardbeschichtung, die die Freisetzung des Wirkstoffs steuert. Derartige Retardbeschichtungen sind im Stand der Technik bekannt, und es kann beispielsweise wiederum auf die EP-A 0 271 193, EP-A 0 553 392 oder auf die WO 2009/059701 verwiesen werden. Diese Retardbeschichtung kann beispielsweise aus einem wasserunlöslichen Polymer oder aus einem Gemisch mehrerer wasserunlöslicher Polymere bestehen z.B. aus einem Gemisch der Produkte Eudragit RL und RS.

In der Regel weist die Retardbeschichtung aber ein Gemisch aus wasserunlöslichen und wasserlöslichen Komponenten auf. Als geeignete wasserlösliche Komponenten kommen im Prinzip all diejenigen wasserlöslichen Polymere in Betracht, die vorstehend als Bindemittel für die innere Wirkstoffschicht genannt wurden. Besonders bevorzugt wird als wasserlösliche Komponente z.B. Hydroxypropylmethylcellulose oder eine andere wasserlösliche Cellulose oder Polyvinylpyrrolidon oder ein ähnliches Material verwendet. Als wasserunlösliche Komponente kann beispielsweise ein Wachs alleine oder im Gemisch mit einem Fettalkohol, wasserunlösliche Cellulose, insbesondere Ethylcellulose oder ein geeignetes Polymethacrylat, beispielsweise ein Produkt der Eudragit^{®}-Reihe verwendet werden. Derartige Materialien sind bekannt und neben den vorstehend genannten Druckschriften beispielsweise auch in der EP-A 0 722 730 beschrieben. Auch die Vermischung der wasserunlöslichen Komponente mit der wasserlöslichen Komponente erfolgt wie im Stand der Technik bekannt. Die auf der Wirkstoffschicht aufgebrachte Retardbeschichtung kann weitere übliche pharmazeutisch verträgliche Hilfs- und Zusatzstoffe wie Farbstoffe, Weichmacher wie Dibutylsebacat, etc. enthalten.

Durch Verwendung der erfindungsgemäßen Teilchen wird ein vorteilhaftes Freisetzungsprofil und eine vorteilhafte Freisetzung des Hydromorphons erreicht.

Die Wirkstoffschicht hat bevorzugt eine Dicke im Bereich von 5 µm bis 200 µm, stärker bevorzugt im Bereich von 5 µm bis 100 µm.

Die Extrusionspellets haben bevorzugt einen Durchmesser im Bereich von 0,2 mm bis 2,6 mm, insbesondere im Bereich von 0,4 mm bis 1,8 mm auf.

Die Retardbeschichtung hat bevorzugt eine Dicke im Bereich von 5 µm bis 200 µm, stärker bevorzugt im Bereich von 5 µm bis 100 µm.

Die erfindungsgemäßen Teilchen haben vorzugsweise einen Durchmesser im Bereich von 200 µm bis 3000 µm, stärker bevorzugt im Bereich von 500 µm bis 2000 µm. Erfindungsgemäß ist es möglich, dass die Teilchen neben den beschriebenen Schichten noch weitere Schichten aufweisen. Beispielsweise können das inerte Pellet und die Wirkstoffschicht, die Wirkstoffschicht und die Retardbeschichtung oder auch das Extrusionspellet und die Retardbeschichtung noch jeweils durch eine Zwischenschicht getrennt sein. Ferner können auf der Retardbeschichtung noch weitere Beschichtungen vorhanden sein. Die Zusammensetzung derartiger Zwischenschichten bzw. äußeren Beschichtungen ist dem Fachmann bekannt, sie bestehen beispielsweise aus einem Bindemittel wie insbesondere einem wasserlöslichen Cellulosepolymer und gegebenenfalls üblichen pharmazeutisch unbedenklichen Hilfs- und Zusatzstoffen. Wesentlich ist, dass diese zusätzlichen Schichten die Freisetzungseigenschaften der erfindungsgemäßen Teilchen nicht negativ beeinflusst. Erfindungsgemäß bevorzugt enthalten die wirkstoffhaltigen Teilchen jedoch keine weiteren Schichten und bestehen z.B. aus dem inerten Pellet, der Wirkstoffschicht und der Retardbeschichtung bzw. aus dem Extrusionspellet und der Retardbeschichtung.

Erfindungsgemäß ist es ebenfalls möglich, allerdings nicht bevorzugt, dass die Teilchen auch noch eine äußere Wirkstoffschicht aufweisen, wie dies in der WO 2009/059701 beschrieben ist.

Die erfindungsgemäßen Teilchen können direkt oder mit üblichen pharmazeutisch verträglichen Hilfs- und Zusatzstoffen zu einem Arzneimittel zur oralen Verabreichung verarbeitet werden. Bevorzugt wird das erfindungsgemäße Arzneimittel zunächst in eine Einheitsdosisform überführt, die zur oralen Verabreichung geeignet und üblich ist. Geeignete Einheitsdosisformen sind beispielsweise Sachets, Stickpacks, Kapseln oder Tabletten. Bevorzugt sind erfindungsgemäß Kapseln, wobei unter Kapseln insbesondere Hypromellose Kapseln verstanden werden. Bevorzugt enthält eine Kapsel 10 bis 1200 mg Teilchen, stärker bevorzugt 20 bis 750 mg an erfindungsgemäßen Teilchen.

Hierzu können die erfindungsgemäßen Teilchen beispielsweise direkt und ohne weitere Hilfs- und Zusatzstoffe zum Beispiel in ein Sachet oder eine Kapsel überführt werden, oder sie können zunächst mit geeigneten Hilfs- und Zusatzstoffen gemischt und anschließend beispielsweise in ein Sachet oder eine Kapsel abgefüllt oder zu einer Tablette verpresst werden. Geeignete Hilfs- und Zusatzstoffe sind dem Fachmann bekannt und beispielsweise im Europäischen Arzneibuch beschrieben. Mögliche Hilfsstoffe sind beispielsweise Sprengmittel, Trennmittel, Füllstoffe, Bindemittel, Zusätze zur Verbesserung der Fließfähigkeit, Schmiermittel, Fließregulierungsmittel und/oder Tenside.

Selbstverständlich können auch Geschmacksstoffe, Färbemittel und weitere Hilfsmittel in dem erfindungsgemäßen Arzneimittel ebenfalls vorhanden sein. Bevorzugt enthalten die erfindungsgemäßen Arzneimittel, wenn es sich um Tabletten handelt, neben den erfindungsgemäßen wirkstoffhaltigen Teilchen noch mindestens einen Füllstoff, stärker bevorzugt mindestens einen Füllstoff und mindestens ein Sprengmittel, noch stärker bevorzugt mindestens einen Füllstoff, mindestens ein Sprengmittel und mindestens ein Bindemittel. Bevorzugt sind ebenfalls noch Schmiermittel und Gleitmittel vorhanden.

Als Bindemittel können die gleichen Bindemittel genannt werden wie sie vorstehend im Zusammenhang mit der inneren wirkstoffhaltigen Schicht offenbart wurden.

Geeignete Füllstoffe sind beispielsweise Laktose, wobei modifizierte Laktose oder wasserfreie (NF) Laktose genannt werden kann, Stärke, insbesondere modifizierte (pregelatinisierte) Stärke, native Stärke oder Gemische von beidem, Calciumphosphat, insbesondere dibasisches, ungemahlenes dibasisches und wasserfreies dibasisches Calciumphosphat, Cellulosederivate, Cellulose, insbesondere mikrokristalline Cellulose, Mannit, Sorbit, etc.

Selbstverständlich können Gemische verschiedener Füllstoffe verwendet werden.

Geeignete Sprengmittel sind beispielsweise Polyvinylpolypyrrolidon (PVPP), Agar, Kartoffelstärke, Formaldehyd-Casein, Natriumcarboxymethylamylopektin, Bentonit, Natriumalginat, Natriumcarboxymethylcellulose, quervernetztes PVP, hochdisperses Siliziumdioxid oder auch Trockenpektin. Wie bei den Bindemitteln und bei den Füllstoffen können selbstverständlich auch bei den Sprengmitteln Gemische verschiedener Sprengmittel verwendet werden.

Geeignete Fließreguliermittel sind erfindungsgemäß bekannt, hierbei kann es sich beispielsweise um "Gleitol", Talk, kolloidales Siliziumdioxid, gefälltes Silica, Calciumstearat, Magnesiumstearat, Stearinsäure, Laurylsäure, Stearylalkohol, Palmitinsäure, Beheninsäure, Caprinsäure, Carbowax^{®} oder Aerosil^{®} handeln.

Auch geeignete Schmiermittel sind dem Fachmann bekannt, wobei viele Verbindungen, die als Fließregulierungsmittel geeignet sind, auch als Schmiermittel verwendet werden können. Geeignete Schmiermittel sind beispielsweise Calciumstearat, Beheninsäure, Stearinsäure, Aluminiumstearat, Stearylalkohol, Natriumstearylfumarat, hydriertes Rizinusöl, Palmitinsäure, Cetylalkohol, Talk, Magnesiumstearat, Myoistinsäure, Lanette^{®} O, Laurylsäure, entfettetes Milchpulver, Gleitol, Talkumin, Caprinsäure, Bolus Alba, Stärke und Polyethylenglykole, wie Carbowax^{®} 6000.

Die Herstellung der erfindungsgemäßen Teilchen erfolgt auf übliche Art und Weise.

Bei der Herstellung der Ausführungsform, bei der ein inertes Pellet mit einer Wirkstoffschicht und einer Retardschicht versehen wird, werden zunächst die Bestandteile der Wirkstoffschicht, z.B. Hydromorphon oder ein pharmazeutisch verträgliches Salz oder Solvat davon, ein Antioxidationsmittel, ein Bindemittel und gegebenenfalls, weitere pharmazeutisch verträgliche Hilfs- und Zusatzstoffe in ein geeignetes Lösemittel eingebracht. Hierbei kann zunächst das Hydromorphon oder das pharmazeutisch verträgliche Salz im Gemisch mit einem Antioxidationsmittel zu dem Lösemittel gegeben werden und anschließend das Bindemittel und gegebenenfalls, weitere pharmazeutisch verträgliche Hilfs- und Zusatzstoffe zugegeben werden. Alternativ können zunächst das Bindemittel und gegebenenfalls, weitere pharmazeutisch verträgliche Hilfs- und Zusatzstoffe in ein Lösemittel eingetragen werden, gefolgt von der Zugabe von Hydromorphon und einem Antioxidationsmittel. Desweiteren können Hydromorphon, ein Antioxidationsmittel, ein Bindemittel und gegebenenfalls, weitere pharmazeutisch verträgliche Hilfs- und Zusatzstoffe gleichzeitig zu dem Lösemittel zugegeben werden. Das Gemisch wird anschließend homogenisiert. Das Lösemittel liegt hier bevorzugt im Überschuss vor.

Bevorzugt sind alle Bestandteile in dem Lösemittel gelöst, so dass eine Lösung entsteht, die als Sprühlösung verwendet wird. Es ist allerdings auch möglich, dass ein oder mehrere Bestandteile nicht oder nicht vollständig in dem Lösemittel löslich sind. In diesem Fall entsteht eine Suspension, die als Sprühsuspension verwendet wird.

Der pH Wert der erhaltenen Sprühlösung bzw. Sprühsuspension kann anschließend gegebenenfalls durch eine geeignete Säurezugabe auf pH 1,5 bis pH 5,5 abgesenkt werden. Als Säure eignet sich hierfür jede pharmazeutisch verträgliche Säure bzw. jede Säure, die bei der Neutralisation des Hydromorphons ein pharmazeutisch verträgliches Salz ergibt. Beispielsweise eignen sich hierfür Salzsäure, Zitronensäure und Essigsäure.

Als geeignete Lösemittel für die Herstellung der Sprühlösung bzw. Sprühsuspension eignen sich alle pharmazeutisch verträglichen und ausreichen flüchtigen Lösemittel, die Hydromorphon oder ein pharmazeutisch verträgliches Salz davon in einem ausreichendem Masse lösen. Als ein für diesen Zweck besonders bevorzugtes Lösemittel wird Wasser, insbesondere demineralisiertes Wasser verwendet.

In einem separaten Arbeitsschritt wird die Sprühlösung bzw. Sprühsuspension in einem Wirbelschichtgerät auf die inerten Pellets gesprüht. Die bevorzugte Prozesstemperatur bei diesem Arbeitsschritt liegt zwischen 30°C und 60°C, besonders bevorzugt zwischen 35°C und 50°C.

Die Beschichtung der Neutralpellets mit der Wirkstoffschicht kann in üblichen Wirbelschichtgeräten z.B. mit einer im unteren Teil angeordneten Sprühdüse, z.B. in einem Ventilus^{®}, Innojet erfolgen. Die erhaltenen wirkstoffhaltigen Kerne werden bevorzugt auf die gewünschte Teilchengröße abgesiebt.

Die Abtrennung der wirkstoffhaltigen Kerne der gewünschten Teilchengröße erfolgt in üblichen Vibrationssieben, z.B. in JEL-FIX 50, J. Engelsmann AG. Bevorzugt erfolgt der Siebvorgang derart, dass die erhaltenen wirkstoffhaltigen Kerne eine Teilchengröße (D50-Wert) im Bereich von 100 bis 3000 µm aufweisen, stärker bevorzugt im Bereich von 500 bis 2000 µm. Zu diesem Zweck kann beispielsweise ein Siebsatz mit Maschenweite von 1,250 mm verwendet werden.

Die Teilchengröße der wirkstoffhaltigen Kerne kann beispielsweise mittels Siebanalyse zum Beispiel unter Verwendung einer Retsch^{®} AS 2000 Vorrichtung bestimmt werden, wobei eine Amplitude von 1,5 mm, ein Intervall von 3 Minuten und eine Probeneinwaage von 50 g als beispielhafte Messbedingung angegeben werden können.

Die wirkstoffhaltigen Kerne werden mit einer Retardbeschichtung versehen. Dies erfolgt bevorzugt in der gleichen Vorrichtung und unter im Wesentlichen gleichen Bedingungen wie das Aufbringen der Wirkstoffschicht auf die inerten Pellets. Bevorzugt wird ein Gemisch aus einem wasserunlöslichen Polymer und einem wasserlöslichen Polymer sowie gegebenenfalls Weichmacher und, gegebenenfalls, weiteren pharmazeutisch verträglichen Hilfs- und Zusatzstoffen in ein geeignetes Lösemittel eingebracht. Als ein geeignetes Lösemittel eignet sich ein übliches pharmazeutisch verträgliches und ausreichend flüchtiges Lösemittel, das diese Verbindungen in einem ausreichenden Masse lösen oder suspendieren kann. Als ein für diesen Zweck besonders bevorzugtes Lösemittel wird Ethanol verwendet.

Die erhaltenen wirkstoffhaltigen Kerne mit der Retardbeschichtung werden bevorzugt auf die gewünschte Teilchengröße gesiebt. Dieser Siebvorgang erfolgt bevorzugt in der gleichen Vorrichtung und unter im Wesentlichen gleichen Bedingungen wie die Abtrennung der wirkstoffhaltigen Kerne ohne Retardbeschichtung.

Bei der erfindungsgemäßen Ausführungsform, bei der der Kern der Teilchen ein Extrusionspellet ist, können die Teilchen auf an sich im Stand der Technik bekannte Art und Weise hergestellt werden. Beispielsweise werden die Bestandteile der Extrusionspellets in einem Intensivmischer vorgelegt und gemischt. Anschließend wir die Masse unter Zugabe einer geeigneten Granulierflüssigkeit, z.B. demineralisiertem Wasser granuliert und dann extrudiert. Alternativ können die Bestandteile der Extrusionspellets, auch z.B. in einem Freifallmischer gemischt und anschließend das Gemisch direkt in einem Extruder befeuchtet und extrudiert werden. In beiden Fällen wurden die noch feuchten Extrakt-Stränge in einem geeigneten Spheronizer auf den gewünschten Formfaktor von ≤ 1.4 gerundet.

Die erhaltenen Extrusionspellets können gesiebt und mit einer Retardbeschichtung versehen werden, wie es im Prinzip bei der Ausführungsform mit dem beschichteten Neutralpellet vorstehend beschrieben wurde. Die entsprechenden Ausführungen gelten auch für die Extrusionspellets.

In einer erfindungsgemäß bevorzugten Ausführungsform werden die erfindungsgemäßen Teilchen dann in Hypromellose Kapseln z.B. der Größe 4 abgefüllt. Die Abfüllung kann mit im Stand der Technik bekannten Abfüllmaschinen, beispielsweise mit Bosch GKF 701, Robert Bosch GmbH erfolgen.

Erfindungsgemäß besonders vorteilhaft ist, dass die erfindungsgemäßen Arzneimittel und insbesondere die erfindungsgemäßen Kapseln eine hervorragende Gleichförmigkeit der

Masse aufweisen, die im Bereich von 95-105 % des durchschnittlichen Gehaltes liegt, bevorzugt im Bereich von 98-102 % des durchschnittlichen Gehalts, besonders bevorzugt im Bereich von 99-101 % vom durchschnittlichen Gehalt. Die Gleichförmigkeit der Masse wird gemäß PH Eur. 6.0 Abschnitt 2.9.5 bestimmt.

Für die Verpackung der erfindungsgemäßen Arzneimittel können beispielsweise Blisterverpackungen aus PVC-PVDC, kindersichere Blisterpackungen aus Aluminium oder Rexam-Flaschen verwendet werden.

Die folgenden Beispiele erläutern die Erfindung.

### Beispiel 1

400 g Hydromorphonhydrochlorid, 20 g Ascorbinsäure und 75 g Hypromellose *(Pharmacoat 606, Fa. Shin-Etsu, apparent viscosity 4,8-7,2 mPas)* werden in 1,7 kg demineralisiertem Wasser aufgelöst und in eine Wirbelschichtapparatur der Firma Innojet eingebracht, die eine unten liegende Sprühdüse aufweist. 7,4 kg Zuckerkügelchen 850-1000 µm (Pharm-a-spheres, Fa. Werner, 90% innerhalb 850 - 1000 µm) werden in die Wirbelschichtapparatur gegeben und auf eine Temperatur von 40 bis 50°C vorgeheizt. In der Apparatur wird die Sprühlösung auf die vorgeheizten Zuckerkügelchen aufgesprüht, wobei die Temperatur im Bereich von 35 bis 50°C gehalten wird, bis die gewünschte Beschichtungsmenge erreicht wurde. Falls erforderlich, kann im Batch-Betrieb gearbeitet werden. Die erhaltenen Kerne werden gesiebt (1,250 mm).

Anschließend wird die Lösung für die Retardbeschichtung der Kerne hergestellt. Hierzu werden 128 g Dibutylsebacat, 192 g Hydroxypropylcellulose (Klucel^{®} EF Pharm, Fa. Hercules) und 640 g Ethylcellulose (Aqualon^{®} N 14, Fa. Hercules, apparent viscosity 11,2-16,8 mPas) nacheinander in 8,6 kg wasserfreiem Ethanol gelöst. Die mit der Wirkstoffschicht beschichteten Kerne werden in der Wirbelschichtapparatur auf eine Temperatur von 40 bis 45°C vorgeheizt. Die Lösung für die Retardbeschichtung wird anschließend auf die vorgeheizten Kerne aufgesprüht, wobei die Temperatur im Bereich von 35 bis 50°C gehalten wird. Hierbei entstehen die erfindungsgemäßen Teilchen. Durch die Schichtdicke kann das Freisetzungsprofil der Teilchen eingestellt werden.

Die derart mit Ethylcellulose beschichteten Teilchen werden durch ein 1,250 mm Sieb gesiebt und in Hypromellose Kapseln eingefüllt.

Die in der folgenden Tabelle 1 aufgeführten Teilchen wurden auf diese Art und Weise hergestellt (alle Zahlenangaben beziehen sich auf mg, sofern nichts anderes ausdrücklich angegeben ist).

**Tabelle 1**

| Bestandteil | Funktion | 1(a) | 1(b) | 1(c) | 1(d) | 1(e) |
|---|---|---|---|---|---|---|
| Kerne | | | | | | |
| Pellets Neut. 850-1000 [mg] | Träger | 133,65 | 133,65 | 185,13 | 74,05 | 296,20 |
| Hydromorphon-HCl [mg] | Wirkstoff | 7,20 | 7,20 | 10,00 | 4,00 | 16,00 |
| Pharmacoat 606 (Hypromeilose) [mg] | Bindemittel | 1,35 | 1,35 | 1,88 | 0,75 | 3,00 |
| Ascorbinsäure [mg] | Antioxidationsmittel | - | - | 0,50 | 0,20 | 1,60 |

| Teilchen | | | | | | |
|---|---|---|---|---|---|---|
| Aqualon^{®} EC N 14 (Ethylcellulose) [mg] | Retardierungsmittel | 11,52 | 11,52 | 16,00 | 6,40 | 25,60 |
| Klucel^{®} EF (Hydroxypropylcellulose) [mg] | Porenbildner | 3,46 | 3,46 | 4,80 | 1,92 | 7,68 |
| Dibutylsebacat [mg] | Weichmacher | 2,30 | 2,30 | 3,20 | 1,28 | 5,12 |

| Kapsel | | | | | | |
|---|---|---|---|---|---|---|
| Gelatine | Kapsel | 1 Stück | | | | |
| HPMC | Kapsel | | 1 Stück | 1 Stück | 1 Stück | 1 Stück |
| Gesamtmenge pro Kapsel [mg] | | 197,5 | 197,5 | 283,5 | 126,6 | 556,4 |

Die Beispiele 1(a) und 1(b) sind Vergleichsbeispiele.

### Beispiel 2

Die Kapseln des Beispiels 1 wurden entweder in Blisterverpackungen (Alu-/PVC-/PVDC-Blister bzw. Duplexblister) oder in eine Rexam-Flasche mit Trockenmittel abgefüllt und einem Lagertest unter Stressbedingungen unterzogen. Die Ergebnisse sind in der folgenden Tabelle 2 zusammengefasst. Alle Zahlenangaben sind in Prozent an Wirkstoff, bezogen auf 100% Wirkstoffgehalt.

**Tabelle 2**

| Beispiel | Verpackung | T/rH | T/rH | T/rH | Lagerzeit | Startwert |
|---|---|---|---|---|---|---|
| | | 25%/60% | 30°C/65% | 40°C/75% | | |
| 1(a) | Duplex-Blister | 96,99 | 96,37 | 95,07 | 1 Monat | 99,69 |
| | | 98,20 | 97,60 | 95,66 | 3 Monate | |
| 1(a) | Rexam-Flasche | 98,16 | 97,82 | 98,05 | 1 Monat | 99,69 |
| 1(a) | Alu-/PVC-/PVDC-Blister | 96,76 | 97,48 | 94,79 | 1 Monat | 99,69 |
| 1(b) | Rexam-Flasche | 98,02 | 97,93 | 93,38 | 1 Monat | 99,69 |
| 1(b) | Alu-/PVC-/PVDC-Blister | 98,30 | 97,31 | 95,55 | 1 Monat | 99,69 |
| 1(c) | Duplex-Blister | 102,56 | 104,27 | 101,89 | 1 Monat | 103,42 |
| | | 102,85 | 104,28 | 103,41 | 3 Monate | |
| | | 105,43 | 103,20 | 100,85 | 6 Monate | |
| 1(d) | Rexam-Flasche | 101,7 | 102,3 | 99,6 | 6 Monate | 102,7 |
| | | 100,6 | 101,1 | 100,2 | 9 Monate | |
| 1(d) | Alu-/PVC-/PVDC-Blister | 98,4 | - | 100,6 | 3 Monate | 98,7 |
| | | 100,7 | - | 101,1 | 18 Monate | |

Weiterhin zeigte sich, dass die Proben, bei denen der Wirkstoff nicht mit einem Antioxidationsmittel formuliert wurde, nach der Lagerung über 1 Monat schon einen Gehalt an unbekannten Verunreinigungen aufweisen, der von vielen nationalen Arzneimittelzulassungsbehörden als bedenklich eingestuft wird, und zwar insbesondere bei einer Lagerung über 40°C und 75% Luftfeuchtigkeit. Außerdem trat bei den Proben ohne Antioxidationsmittel bei längerer Lagerzeit häufig eine leichte Gelbfärbung des Arzneimittels auf.

Insgesamt wird aus den vorstehenden Daten ersichtlich, dass die erfindungsgemäßen Arzneimittel, bei denen die Wirkstoffschicht der wirkstoffhaltigen Kerne den Wirkstoff im Gemisch mit einem Antioxidationsmittel enthalten, eine wesentlich verbesserte Lagerstabilität aufweist, und zwar über einen Zeitraum von bis zu 18 Monaten, auch unter Stressbedingungen. Dagegen tritt bei Arzneimitteln, die kein Antioxidationsmittel enthalten, in der Regel schon nach einem Monat ein erheblicher Abbau des Wirkstoffs auf. Dies ist überraschend insbesondere, da sich im Stand der Technik bei festen Arzneiformen keinerlei Hinweis auf eine mögliche Empfindlichkeit des Hydromorphons gegenüber Luftsauerstoff oder auf die Verwendung von Antioxidationsmittein zur Stabilisierung des Hydromorphons findet.

### Beispiel 3

Die Bestandteile für Extrusionspellets, wie sie in Tabelle 3 angegeben sind wurden in einem Intensivmischer vorgelegt und 5 Minuten gemischt, bis eine homogene Mischung entstanden war. Die Gemische wurden mit ausreichend demineralisiertem Wasser granuliert und anschließend extrudiert. Die noch feuchten Extrudat-Stränge wurden in einen Spheronizer eingebracht und auf einen Formfaktor von ≤ 1.4 gerundet.

Aus den in Tabelle 3 angegebenen Bestandteilen für die Retardschicht wurde entsprechend Beispiel 1 eine Beschichtungslösung hergestellt und die Extrusionspellets wurden analog der Vorschrift des Beispiels 1 damit beschichtet. Die erhaltenen Teilchen wurden getrocknet, in die HPMC-Kapseln abgefüllt und wie in Tabelle 3 angegeben verpackt.

**Tabelle 3**

| | **Beispiel 3a** | **Beispiel 3b** | **Beispiel 3c** | **Beispiel 3d** | **Beispiel 3e** |
|---|---|---|---|---|---|
| **Extrusions-Pellets** | | | | | |
| Hydromorphon HCl [mg] | 4,00 | 4,00 | 4,00 | 4,00 | 4,00 |
| Microcrystalline Cellulose [mg] | 62,80 | 50,80 | 50,80 | 62,80 | 51,00 |
| Hypromellose [mg] | - | 4,00 | - | - | 4,00 |
| Povidon [mg] | - | - | 4,00 | - | - |
| Hyprolose [mg] | 4,00 | - | - | 4,00 | - |
| Ascorbinsäure [mg] | 0,20 | 0,20 | 0,20 | 0,20 | - |
| Mannit [mg] | - | 20,00 | - | - | 20,00 |
| L-HPC [mg] | 8,00 | - | - | - | - |
| Sucrose [mg] | - | - | 20,00 | - | - |
| Kollidon CL [mg] | - | - | - | 8,00 | - |
| *Gesamtgewicht (Trockensubstanz)* [mg] | *79,00* | *79,00* | *79,00* | *79,00* | *79,00* |

| **Beschichtete Teilchen** | | | | | |
|---|---|---|---|---|---|
| Ethylcellulose (12 - 16 cP) [mg] | 6,40 | 6,40 | 6,40 | 6,40 | 6,40 |
| HPC (300 - 600 cP) [mg] | 1,92 | 1,92 | 1,92 | 1,92 | 1,92 |
| Dibutylsebacat, NF [mg] | 1,28 | 1,28 | 1,28 | 1,28 | 1,28 |
| Ethanol A15 [mg] | 86,50 | 86,50 | 86,50 | 86,50 | 86,50 |
| *Gesamtgewicht (Trockensubstanz)* [mg] | *88,60* | *88,60* | *88,60* | *88,60* | *88,60* |

| **HPMC Kapseln** | | | | | |
|---|---|---|---|---|---|
| Größe 4, Ivory (Head) White (Body) | 1 unit | 1 unit | 1 unit | 1 unit | 1 unit |

| **Verpackung** | | | | | |
|---|---|---|---|---|---|
| Rexam 100 ml white | qs | qs | qs | qs | qs |
| Rexam CRC 3.4g dessicant | qs | qs | qs | qs | qs |
| PVC 250 µm PVDC 90 g/m² (Ineos) | qs | qs | qs | qs | qs |
| Alu 20 µm peel-of (Alcan) | qs | qs | qs | qs | qs |

Beispiel 3(e) ist ein Vergleichsbespiel.

Die erfindungsgemäßen Teilchen zeigten eine hervorragende Lagerstabilität.

## Patentansprüche

1. Wirkstoffhaltiges Teilchen mit retardierter Wirkstofffreisetzung, umfassend einen Kern, der den Wirkstoff enthält und eine Retardbeschichtung, die die Freisetzung des Wirkstoffs retardiert, wobei es sich bei dem Wirkstoff um Hydromorphon oder ein Salz oder Solvat davon handelt und wobei der Kern das Hydromorphon oder das pharmazeutisch verträgliche Salz oder das Solvat davon im Gemisch mit einem Antioxidationsmittel enthält, wobei es sich bei dem Antioxidationsmittel um Ascorbinsäure oder ein Salz oder einen Ester davon handelt.

2. Wirkstoffhaltiges Teilchen nach Anspruch 1, wobei das Teilchen einen Gehalt an Hydromorphon oder einem pharmazeutisch verträglichen Salz davon oder einem Solvat davon im Bereich von 2 Gew.-% bis 20 Gew.-% bezogen auf das Gewicht des Teilchens aufweist.

3. Wirkstoffhaltiges Teilchen nach Anspruch 1 oder 2, wobei das Teilchen keinen Opioidantagonisten enthält.

4. Wirkstoffhaltiges Teilchen nach einem der Ansprüche 1 bis 3, wobei die Retardbeschichtung Ethylcellulose enthält.

5. Wirkstoffhaltiges Teilchen nach einem der Ansprüche 1 bis 4, wobei das Gewichtsverhältnis von Hydromorphon bzw. dem Salz oder dem Solvat davon zu dem Antioxidationsmittel im Bereich von 40:1 bis 5:1 liegt.

6. Wirkstoffhaltiges Teilchen nach einem der Ansprüche 1 bis 5, bei dem der Kern, der den Wirkstoff enthält,
a) ein inertes Pellet und
b) eine auf dem inerten Pellet aufgebrachte Wirkstoffschicht umfasst, die den Wirkstoff im Gemisch mit dem Antioxidationsmittel aufweist.

7. Wirkstoffhaltiges Teilchen nach Anspruch 6, bei dem die Retardbeschichtung direkt auf der Wirkstoffschicht aufgebracht ist.

8. Wirkstoffhaltiges Teilchen nach einem der Ansprüche 6 oder 7, wobei die Wirkstoffschicht aus Hydromorphonhydrochlorid, Hydroxypropylmethylcellulose und Ascorbinsäure besteht.

9. Wirkstoffhaltiges Teilchen nach einem der Ansprüche 6 bis 8, wobei der Gehalt an Antioxidationsmittel in der auf dem inerten Pellet aufgebrachten Wirkstoffschicht im Bereich von 0,01 Gew.-% bis 30 Gew.-% bezogen auf das Gewicht des Teilchens liegt.

10. Wirkstoffhaltiges Teilchen nach einem der Ansprüche 1 bis 5, wobei es sich bei dem Kern, der den Wirkstoff enthält, um ein Extrusionspellet handelt, in dem der Wirkstoff im Gemisch mit einem Sphäronisierungsmittel und dem Antioxidationsmittel vorliegt.

11. Wirkstoffhaltiges Teilchen nach Anspruch 10, bei dem das Sphäronisierungsmittel mikrokristalline Cellulose ist.

12. Wirkstoffhaltiges Teilchen nach einen der Ansprüche 10 oder 11, das einen Formfaktor von ≤ 1,4 aufweist.

13. Arzneimittel zur oralen Verabreichung mit mindestens einem wirkstoffhaltigen Teilchen nach einem der Ansprüche 1 bis 12.

14. Arzneimittel nach Anspruch 13 mit einem Gehalt an Hydromorphon oder dem Salz oder Solvat davon im Bereich von 2 mg bis 40 mg.

15. Verfahren zur Herstellung eines wirkstoffhaltigen Teilchens nach einem der Ansprüche 6 bis 9, bei dem
(i) ein inertes Pellet mit einer wirkstoffhaltigen Schicht beschichtet wird,
(ii) das so erhaltene Produkt getrocknet wird und
(iii) das getrocknete Produkt mit einer Retardbeschichtung beschichtet wird.

16. Verfahren zur Herstellung eines wirkstoffhaltigen Teilchens nach einem der Ansprüche 10 bis 12, bei dem
(i) ein Gemisch umfassend den Wirkstoff, das Sphäronisierungsmittel und das Antioxidationsmittel extrudiert,
(ii) in einem Spheronizer gerundet und anschließend
(iii) mit der Retardbeschichtung beschichtet wird.

17. Arzneimittel nach Anspruch 13 oder 14 zur Verwendung zur Behandlung chronischer Schmerzen.

## Claims

1. An active ingredient-containing particle with retarded release of active ingredient, comprising a core which contains the active ingredient, and a retarded-release coating that retards the release of the active ingredient, wherein said active ingredient is hydromorphone or a salt or solvate thereof, and wherein the core contains the hydromorphone or the pharmaceutically acceptable salt or the solvate thereof in a mixture with an antioxidant, wherein the antioxidant is ascorbic acid or a salt or an ester thereof.

2. The active ingredient-containing particle according to claim 1, wherein the particle has a content of hydromorphone or a pharmaceutically acceptable salt thereof or a solvate thereof in the range of 2% by weight to 20% by weight based on the weight of the particle.

3. The active ingredient-containing particle according to claim 1 or 2, wherein the particle does not contain an opioid antagonist.

4. The active ingredient-containing particle according to any one of claims 1 to 3, wherein the retarded-release coating contains ethyl cellulose.

5. The active ingredient-containing particle according to any one of claims 1 to 4, wherein the weight ratio of hydromorphone or the salt or the solvate thereof, respectively, to the antioxidant is in the range of 40:1 to 5:1.

6. The active ingredient-containing particle according to any one of claims 1 to 5, wherein the core containing the active ingredient comprises
a) an inert pellet and
b) an active ingredient layer applied to the inert pellet which has the active ingredient in a mixture with the antioxidant.

7. The active ingredient-containing particle according to claim 6, wherein the retarded-release coating is directly applied to the active ingredient layer.

8. The active ingredient-containing particle according to any one of claims 6 or 7, wherein the active ingredient layer consists of hydromorphone hydrochloride, hydroxypropyl methylcellulose and ascorbic acid.

9. The active ingredient-containing particle according to any one of claims 6 to 8, wherein the content of antioxidant in the active ingredient layer applied to the inert pellet is in the range of 0.01 % by weight to 30% by weight based on the weight of the particle.

10. The active ingredient-containing particle according to any one of claims 1 to 5, wherein the core containing the active ingredient is an extrusion pellet in which the active ingredient is present in a mixture with a spheronizing agent and the antioxidant.

11. The active ingredient-containing particle according to claim 10, wherein the spheronizing agent is microcrystalline cellulose.

12. The active ingredient-containing particle according to any one of claims 10 or 11 which has a form factor of ≤ 1.4.

13. A medicinal product for the oral administration with at least one active ingredient-containing particle according to any one of claims 1 to 12.

14. The medicinal product according to claim 13 with a content of hydromorphone or the salt or solvate thereof in the range of 2 mg to 40 mg.

15. A method for the production of an active ingredient-containing particle according to any one of claims 6 to 9, wherein
(i) an inert pellet is coated with an active ingredient-containing layer,
(ii) the thus obtained product is dried; and
(iii) the dried product is coated with a retarded-release coating.

16. The method for the production of an active ingredient-containing particle according to any one of claims 10 to 12, wherein
(i) a mixture comprising the active ingredient, the spheronizing agent, and the antioxidant is extruded,
(ii) made round in a spheronizer and subsequently
(iii) coated with a retarded-release coating.

17. The medicinal product according to claim 13 or 14 for use in the treatment of chronic pain.

## Revendications

1. Particule contenant une substance active, à libération retardée de la substance active, comprenant un noyau qui contient la substance active et un revêtement de retardement qui retarde la libération de la substance active, dans laquelle la substance active est l'hydromorphone ou un sel ou solvate de celle-ci et dans laquelle le noyau contient l'hydromorphone ou le sel pharmaceutiquement compatible ou le solvate de celle-ci en mélange avec un antioxydant, l'antioxydant étant l'acide ascorbique ou un sel ou un ester de celui-ci.

2. Particule contenant une substance active selon la revendication 1, dans laquelle la particule présente une teneur en hydromorphone ou un sel pharmaceutiquement compatible ou un solvate de celle-ci dans la plage allant de 2 % en poids à 20 % en poids, par rapport au poids de la particule.

3. Particule contenant une substance active selon la revendication 1 ou 2, dans laquelle la particule ne contient pas d'antagonistes d'opioïdes.

4. Particule contenant une substance active selon l'une quelconque des revendications 1 à 3, dans laquelle le revêtement de retardement contient de l'éthylcellulose.

5. Particule contenant une substance active selon l'une quelconque des revendications 1 à 4, dans laquelle le rapport en poids entre l'hydromorphone ou le sel ou le solvate de celle-ci et l'antioxydant se situe dans la plage allant de 40:1 à 5:1.

6. Particule contenant une substance active selon l'une quelconque des revendications 1 à 5, dans laquelle le noyau qui contient la substance active comprend
a) une pastille inerte et
b) une couche de substance active appliquée sur la pastille inerte, qui comprend la substance active en mélange avec l'antioxydant.

7. Particule contenant une substance active selon la revendication 6, dans laquelle le revêtement de retardement est appliqué directement sur la couche de substance active.

8. Particule contenant une substance active selon l'une quelconque des revendications 6 ou 7, dans laquelle la couche de substance active est constituée de chlorhydrate d'hydromorphone, d'hydroxypropyl-méthylcellulose et d'acide ascorbique.

9. Particule contenant une substance active selon l'une quelconque des revendications 6 à 8, dans laquelle la teneur en antioxydant dans la couche de substance active appliquée sur la pastille inerte se situe dans la plage allant de 0,01 % en poids à 30 % en poids, par rapport au poids de la particule.

10. Particule contenant une substance active selon l'une quelconque des revendications 1 à 5, dans laquelle le noyau qui contient la substance active est une pastille extrudée dans laquelle la substance active se présente en mélange avec un agent de sphéronisation et l'antioxydant.

11. Particule contenant une substance active selon la revendication 10, dans laquelle l'agent de sphéronisation est la cellulose microcristalline.

12. Particule contenant une substance active selon l'une quelconque des revendications 10 ou 11, qui présente un facteur de forme ≤ 1,4.

13. Médicament pour administration orale contenant au moins une particule contenant une substance active selon l'une quelconque des revendications 1 à 12.

14. Médicament selon la revendication 13 ayant une teneur en hydromorphone ou son sel ou solvate dans la plage allant de 2 mg à 40 mg.

15. Procédé de fabrication d'une particule contenant une substance active selon l'une quelconque des revendications 6 à 9, selon lequel
(i) une pastille inerte est revêtue avec une couche contenant une substance active,
(ii) le produit ainsi obtenu est séché et
(iii) le produit séché est revêtu avec un revêtement de retardement.

16. Procédé de fabrication d'une particule contenant une substance active selon l'une quelconque des revendications 10 à 12, selon lequel
(i) un mélange comprenant la substance active, l'agent de sphéronisation et l'antioxydant est extrudé,
(ii) arrondi dans un sphéroniseur, puis
(iii) revêtu avec un revêtement de retardement.

17. Médicament selon la revendication 13 ou 14, destiné à une utilisation pour le traitement de la douleur chronique.
